# EUROPEAN PATENT SPECIFICATION

(11) **EP 1 169 074 B1**
(45) Date of publication and mention of the grant of the patent: **25.01.2006**
(21) Application number: 00920994.1
(22) Date of filing: 31.03.2000
(51) Int. Cl.: A61M 5/315

(54) **PROCESS FOR DETERMINING THE SUITABILITY OF A SEALING MEMBER FOR A STORAGE CONTAINER FOR WEAKLY ACIDIC SOLUTION FORMULATIONS CONTAINING HUMAN GROWTH HORMONE**
VERFAHREN ZUR FESTSTELLUNG DER EIGNUNG EINES DICHTUNGSELEMENTS FÜR EINEN AUFBEWAHRUNGSBEHÄLTER FÜR SCHWACH SAUERE LÖSUNGSFORMELN ENTHALTEND MENSCHLICHES WACHSTUMHORMON
PROCEDE DE DETERMINATION DE L'APTITUDE D'UN ELEMENT D'ETANCHEITE POUR UN RECIPIENT DE STOCKAGE D'UNE PREPARATION DE SOLUTION FAIBLEMENT ACIDE CONTENANT L'HORMONE DE CROISSANCE HUMAINE

(30) Priority: 02.04.1999 JP 9644399
(43) Date of publication of application: 09.01.2002
(73) Proprietor: SUMITOMO PHARMACEUTICALS COMPANY, LIMITED, Osaka 541-8510 (JP)
(72) Inventor: MORITA, Shigetoshi, Higashinada-ku, Koube City 658-0032 (JP); TANAKA, Katsumi, Takatsuki City, Osaka-ku 569-0857 (JP); YOSHIMOTO, Atsushi, Takarazuka City, Hyougo Prefc. 665-0046 (JP)
(74) Representative: Stuart, Ian Alexander
(86) International application number: PCT/IB2000/000608
(87) International publication number: WO 2000/059562

(56) References cited:
- EP-A- 0 148 426
- US-A- 4 977 027
- US-A- 5 064 083
- DATABASE WPI Section Ch, Week 199317 Derwent Publications Ltd., London, GB; Class A12, AN 1993-140154 XP002146187 -& JP 05 077846 A (POLYTECH DESIGN KK), 30 March 1993 (1993-03-30)
- DATABASE WPI Section Ch, Week 199624 Derwent Publications Ltd., London, GB; Class B04, AN 1996-235992 XP002146188 -& JP 08 092125 A (NIPPON CHEM RES KK), 9 April 1996 (1996-04-09) cited in the application

## Description

### TECHNICAL FIELD

The present invention relates to a process for determining whether a sealing member is of suitable material for use in a storage container for a weakly acidic solution formulation containing human growth hormone, e.g. in an injection cartridge therefor.

### BACKGROUND ART

Human growth hormone (sometimes referred to as "hGH" below) is a single-chain polypeptide hormone composed of 191 amino acid residues, hGH can undergo decomposition by a number of routes, for example, by deamidation, flocculation, precipitation, oxidation of methionine residues and proteolysis. In order to avoid such decomposition reactions, hGH has conventionally been formulated and sold in freeze-dried form. However, recent years have seen a rising demand for the development of solution formulations for dinical reasons such as in order to improve the compliance of patients by simplifying the method of use, and various such formulations have been announced (see, e.g, PCT Application, Japanese-Language Publication No. Hei 7-809719; Japanese Patent Application, First Publication No. Hel 8-92125).

These solution formulations employ a weakly acidic buffer solution with a pH (pH 6-7) slightly less than the weakly alkaline physiological pH, pH 7-7.5, which has been conventionally employed in freeze-dried formulations. This is because slight alkalinity may cause deamidation of the hGH during storage as a solution. However, with slight acidity of pH 6-7, hGH may tend to precipitate, so that the addition of surfactants has been necessitated for long-term storage. Additionally, the present inventors have observed that even when surfactants are added, precipitation or nebulation of the hGH can occur during long-term storage of the hGH solution depending on the conditions, and the cause of this phenomenon has conventionally been completely unexplained.

On the other hand, since the rubber stoppers or rubber plungers used In Injection-type solution formulations are in contact with the solution for a long time in comparison to the case where used in the container of a freeze-dried solution, problems in quality caused by the rubber stopper material can often occur. Whereas examples of problems associated With rubber stoppers include contaminants adhering to the rubber stopper, coring and sticking, a particular problem for solution formulations is the effect of elutes from the rubber stopper on the quality of the pharmaceutical agent. Rubber stoppers have very complicated properties both chemically and physical, and various types of elute substances from rubber stoppers are known. These are, for example, reported by L. Gramiccioni *et al*. (*Chromatographia*, 28 ('89) 545-550). However, it has yet to be examined which of the elute substances from rubber stoppers have what type of effects on a hGH solution formulation, particularly weakly acidic solution formulations, and there have been no such reports as far as the inventors are aware.

Therefore, the present inventors performed diligent research in this regard, as a result of which they discovered that the formulation container, particularly the material of the rubber stopper is an important factor in the stable storage of hGH solution formulations. That is, they discovered that metal ions dissolve from the rubber stopper during long-term storage and form conjugates with the hGH. Based on this discovery, they found that it is necessary to use a rubber stopper in which the elution of metal ions (especially zinc ions and/or aluminum ions) under certain conditions is below a standard amount in order to prevent degradations of the quality of the hGH solution formulation, thereby arriving at the present invention.

### DISCLOSURE OF THE INVENTION

The present invention provides a process for determining the suitability of the material of a sealing member as set out in claim 1. The sealing member may be for use in a storage container for a weakly acidic solution formulation containing human growth hormone which comprises a cylindrical container having a first opening and a second opening, and an internal cavity connecting the first opening and second opening; a first sealing member for sealing the first opening; and a second sealing member provided in the internal cavity of the cylindrical container, such as to be capable of moving along the internal cavity while forming a continuous seal in a circumferential direction with an inner well which forms this internal cavity, thereby forming an enclosed space with the first sealing member for containing the weakly acidic solution formulation containing human growth hormone. The second sealing member is composed of a type of rubber having minimal elution of metal ions as determined by the process of the invention. The rubber has a level of elution of metal lons which does not degrade the human growth hormone in the formulation. In particular, the rubber is such that after such a second sealing member is immersed in 1 ml of a buffer solution containing a surfactant and having a pH of 5.5-6.5 and stored while shaking at a temperature of 25 °C for 1 week, the elution rate of polyvalent metal ions in the buffer solution as measured by atomic absorption spectrophotometry is preferably 50 ppm or less.

The first sealing member may also be composed of a type of rubber found suitable by means of the process of the invention, having minimal elution of metal ions. Preferably, the rubber has a level of elution of metal ions which does not degrade the human growth hormone in the formulation. More preferably, the rubber is such that after such a first sealing member is immersed in 1 ml of a buffer solution containing a surfactant and having a pH of 5.5-6.5 and stored while shaking at a temperature of 25 °C for 1 week, the elution rate of polyvalent metal ions in the buffer solution as measured by atomic absorption spectrophotometry is 50 ppm or less.

Preferably a storage container for a weakly acidic solution formulation containing human growth hormone including a sealing member found suitable by the process of the present invention is such that the elution rate of polyvalent metal ions is 20 ppm or less.

The polyvalent metal ions may be zinc ions or aluminum ions.

An injection cartridge for a weakly acidic solution formulation containing human growth hormone including a sealing means which is of suitable material according to the present invention comprises a cylindrical container having a first opening and a second opening, and an intemat cavity connecting the first opening and second opening; a first sealing member for sealing the first opening, having a thickness such as to be capable of being punctured by a syringe needle; and a second sealing member provided in the internal cavity of the cylindrical container, such as to be capable of moving along the internal cavity while forming a continuous seal in a circumferential direction with an inner wall which forms this internal cavity, thereby forming an enclosed space with the first sealing member for containing the weakly acidic solution formulation containing human growth hormone. At least the second sealing member and preferably also the first sealing member is composed of said suitable material whicher a type of rubber having minimal elution of metal ions. Preferably, the rubber has a level of elution of metal ions which does not degrade the human growth hormone in the formulation. More preferably, the rubber is such that after such a second sealing member is immersed in 1 ml of a buffer solution containing a surfactant and having a pH of 5.5-6.5 and stored while shaking at a temperature of 25 °C for 1 week, the elution rate of polyvalent metal ions in the buffer solution as measured by atomic absorption spectrophotometry is 50 ppm or less.

A method for storing a weakly acidic solution containing human grown hormone suitably comprises steps of preparing a cylindrical container having a first opening and a second opening, and an internal cavity connecting the first opening and second opening: providing a rubber stopper in the internal cavity of the cylindrical container, such as to be capable of moving along the internal cavity while forming a continuous seal in a circumferential direction with an inner wall which forms this Internal cavity, thereby forming a space with the first sealing member, fining the space with the weakly acidic solution formulation containing human growth hormone; and sealing the first opening with a cap. The rubber stopper is composed of a type of rubber having minimal elution of metal ions as determined by the process of the invention. Preferably, the rubber has a level of elution of metal ions which does not degrade the human growth hormone in the formulation. More preferably, the rubber is such that after such a rubber stopper is immersed in 1 ml of a buffer solution containing a surfactant and having a pH of 5.5-6.5 and stored while shaking at a temperature of 25 °C for 1 week, the elution rate of polyvalent metal tons in the buffer solution as measured by atomic absorption spectrophotometry is 50 ppm or less.

A polyvalent metal ion chelating agent may be added to the weakly acidic solution formulation containing a human growth hormone.

The terminology such as "buffer solution containing a surfactant" used in the present specification is defined as follows. "Buffer solution containing a surfactant" refers to a solution containing a citric acid-type, phosphoric acid-type, glycine-type or tris-type buffer, an isotonic agent such as sodium chloride, a surfactant such as Polysorbate 80, Polysorbate 20 or Poloxamer 188, and optionally, other preservatives and the like as needed. Polysorbate 20, Poloxamer 188 and the like are preferred as surfactants.

"Rubber stopper or rubber plunger" refers to a rubber stopper for a syringe vial or a plunger used In a cartridge for a convenience-type syringe formulation. That is, a rubber stopper is a sealing plug composed of rubber used for an antiseptic seal after a vial container is filled with hGH. A rubber plunger is a sealing plug composed of rubber used for an antiseptic seal in an hGH solution-filled cartridge used in hGH administration devices.

"hGH" refers to human growth hormone which was brought into practice almost 20 years ago as a treatment for pituitary dwarfism, of which various medical formulations are commercially available. In the present invention, hGH indudes not only hGH proteins from the human pituitary gland (191 amino acids, molecular weight approximately 22.000), but also to human growth hormone equivalents having biologically specific biological activity (e.g. substitution medifications, addition modifications, deletion modifications). Here, biological activity specific to hGH refers mainly to overall growth accelerating activity for causing all human tissues (especially bones) except for the brain to grow mainly during the developmental period, including the effects of accelerating production of bones and cartilage by IGF-I induction, promotion of amino acid intake to cells and protein synthesis, suppression of protein decomposition, promotion of neutral fat metabolism, promotion of sugar metabolism and promotion of electrolyte retention.

"Weakly acidic solution formulation containing hGH' refers to a solution formulation having a buffer with a pH of 5.5-7. and containing hGH as an active ingredient. The appropriate pH range for such an hGH solution formulation Is 5.5-7.0, and has been reported to be more advantageously 6.0 (PCT Application, Japanese Language Publication No. Hel 7-509719).

"Storage container" refers to a fluid storage container such as a vial or cartridge for a syringe as commonly used In the field of pharmaceuticals.

According to the storage container for a weakly acidic solution formulation containing human growth hormone, Injection cartridge therefor and storage method therefor of the present invention employing this type of structure, law levels of nebulation preferably, no nebulation is observed In the storage container containing human growth hormone, thus making it possible to offer an hGH solution formulation which is physically and chemically stable.

### BRIEF DESCRIPTION OF THE DRAWINGS

Fig. 1 is a side view showing a portion of a storage container in cross-section.
Fig. 2 is a perspective view showing the state of use of the storage container shown in Fig. 1.

### BEST MODE FOR CARRYING OUT THE INVENTION

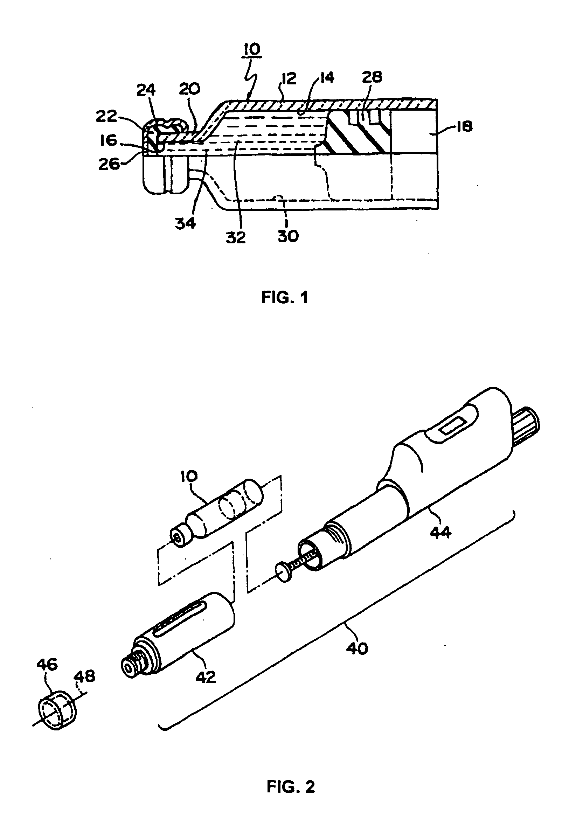

Herebelow, a mode for carrying out the present invention shall be described with reference to the drawings. Fig. 1 shows a storage container. The storage container 10 has a roughly cylindrical container body 12. The container body 12 forms an internal cavity 14, this internal cavity 14 being open at the openings 16, 18 at the ends thereof. In the present embodiment, one end of the container body 12 has a smaller diameter to form a mouth portion 20. The mouth portion 20 has a thin rubber cap 22 and a metallic cap 24 covering this rubber cap 22. As shown in the drawings, these caps 22 and 24 are attached by pressing the cylindrical portion of the metallic cap 24 and the end portion of the tubular portion toward the mouth portion 20 and deforming it. The metallic cap 24 has an opening 26 opposing the opening 16 on roughly the central axis of the container body, such that by passing a needle into this opening 26 and through the rubber cap 22, it is possible to withdraw fluid from the inside.

In the internal cavity 14 of the container body 12, a roughly cylindrical rubber stopper 28 or rubber plunger is inserted from the opening 18 on the other side. The rubber stopper 28 has a slightly larger outer diameter than the inner diameter of the internal cavity 14 of the container body when in a state of withdrawal from the container body 12. Consequently, when the rubber stopper 28 is in a state of insertion into the internal cavity 14 of the container body 14, a continuous seal is formed between the inner wall 30 forming this internal cavity 14 and the outer circumferential surface of the rubber stopper 28, as a result of which an enclosed chamber 32 is formed between the rubber cap 26 and the rubber stopper 28, and a liquid, i.e. human growth hormone solution (weakly acidic solution formulation containing human growth hormone) 34 can be accommodated in this chamber 32.

When sealing human growth hormone solution 34 into the container body 12, the rubber stopper 28 is inserted from the opening 18 with the caps 26 and 28 unattached to the opening 16. Next, human growth hormone solution 34 is injected into the container body 12 from the opening 16. Finally, this opening 16 is covered with the rubber cap 22 and metallic cap 24, and the edge of the tubular portion of the metallic cap 24 is deformed towards the mouth portion 20 to close the seal. Alternatively, the opening 16 is covered with the rubber cap 22 and the metallic cap 24, and the edge of the tubular portion of the metallic cap 24 is deformed towards the mouth portion 20 to close the seal. Next, the human growth hormone solution 34 is injected into the container body 12 through the opening 18. Finally, the rubber stopper 28 is inserted from the opening 18 while compressing to deform.

The human growth hormone solution 34 contained in the storage container 12 having this type of structure is, for example, injected into a patient using the syringe device (administration device) 40 of Fig. 2 offered under the trade name "Pen 100S" from Disetronic. This syringe device 40 is composed of a holder 42 for accommodating the storage container 10 and an actuator 44 coupled to the rear end of this holder 42. Upon use, the storage container 10 is inserted into the holder 42 and the actuator is fitted to the rear end of this holder 42. Additionally, a cap 46 is attached to the front end of the holder 42. This cap 46 is provided with a needle 48 on an end surface, the two tips of this needle 48 protruding respectively from the inside end surface and outside end surface, the end of the needle 48 protruding from the inner end surface puncturing the rubber cap 22. In this state, the actuator 44 is operated, and the rubber stopper 28 of the storage container 12 is pressed. As a result, the human growth hormone solution 34 inside the storage container is delivered through the needle 46.

Herebelow, the rubber stopper of the storage container 12 shall be explained in detail. There are no restrictions as to the material of the rubber stopper as long as it is a material capable of being used in rubber stoppers for medical purposes. Butyl rubber, butyl chloride rubber and butadiene rubber are known as basic elastomers, and any of these may be used. Additionally, while the rubber stopper (or plunger) is used in combination with a vial and injection cartridge, their material and snape are not particularly restricted. Aside from glass which is commonly used, it Is also possible to use, for example, synthetic resins such as polypropylene.

A rubber stopper suitable for the solution storage container is selected by the following experiments.
(1) A buffer solution (pH 6) containing a surfactant is prepared, and 1 ml is put into a glass vial. The above-mentioned solution may optionally include isotonics, stabilizers, preservatives, anti-oxidants, solubilizers and excipients as appropriate. The test conditions may be changed according to the composition, storage conditions and method of use of the hGH solution formulation which is to be used, but in view of the purpose of strictly evaluating the amount of elutes from the rubber stopper, it is undesirable to add agents such as chelating agents which may have an effect on the metal ions.
(2) A single rubber stopper (approximately 1 9) is immersed in the above-described vial, and stored while shaking at 25 °C for one week.
(3) The amount of metal tons which have dissolved into the buffer solution is measured by atomic absorption spectrophotometry.
(4) Suitably, rubber stoppers having an elution rate of 50 ppm or less of polyvalent metal ions, particularly zinc and/or aluminum are selected. Preferably, those with an elution rate of zinc and/or aluminum ions of 20 ppm or less per rubber stopper under the above-given conditions are chosen.
(5) If the rubber stopper material falls to reach the above standards, it can be modified or treated to provide it with properties suitable for the storage container of the present invention by means of surface treatment or the like. For example, by coating the rubber stopper with a fluorine resin laminate, plastic, bulk silicon or other macromolecules by means of commonly known methods, it is possible to prevent the rubber stopper from directly contacting the hGH solution, thereby suppressing the elution of metal ions from the rubber stopper. Rubber stoppers coated by means of such conventional methods should be evaluated by means of the test described in paragraphs (1)-(4), and selected under similar criteria for employment in the container of the present invention.

Unlike a rubber stopper for a vial, a plunger must use a material which is harder (there are usually more additives to the rubber) than that of a simple vial stopper due to the functional property of moving inside the cartridge during use and deciding the dosage delivered. While coatings by bulk silicon or the like which may be scraped off due to friction are not generally held to be preferable for surface treatment, it is possible to have a coating with only a small amount of silicon in order to reduce the friction. Therefore, the plunger which is suitable for carrying out the present invention must clear standards which are more stringent than those of a normal rubber stopper for vials. Specifically, the tests described in paragraphs (1)-(3) should be performed, and those with a zinc and/or aluminum ion concentration of 20 ppm or less should be used.

Herebelow, experiments performed on the rubber stoppers of the storage container shall be explained.

### 1) Method of Analysis

### (i) Metal Ion Content

The quantitative analysis of metal ions in the solution was performed using atomic absorption spectrophotometry according to conventional methods.

### (ii) hGH Content, Polymer Content

Size-exclusion chromatography was performed using a TSK G2000SWXL with 200 mM sodium phosphate (pH 6.8)/0.1% SDS/0.04% Polysorbate 20 as the mobile phase. The flow rate was 0.7 mL/min, and measured at 214 nm.

### (ii) Deamidate Content

Anion exchange chromatography ((HPIEC) was performed using a TSK DEAE 3SW column (0.75 mm × 7.5 cm) at 40 °C with a flow rate of 1.0 mL/min. This column was equilibrated with a 25 mM bis-tris buffer (pH 5.8). Elution was performed using a 40 min gradient of 25 mM bis-tris buffer/0.5 M sodium chloride. Measurements were performed at 280 nm.

### 2) Experimental Method

### (i) Experiment 1 Effects of Metal Ions on hGH

1 mL of a solution formed of 1 mL of a 10 mM citric acid buffer solution (pH 6.0) with 5.0 mg of hGH, 8.77 mg of sodium chloride, 2.5 mg of phenol and 2.0 mg of Polysorbate 20 was antiseptically filled into a glass bottle. A solution in which was dissolved zinc acetate, aluminum chloride, calcium chloride and magnesium chloride was antiseptically added to the above-described sample so as to make the metal ion concentration a standard concentration, and the changes in the solubility state were observed.

### (ii) Experiment 2 Elution of Metal Ions from Rubber Stopper

1 mL of a solution formed of 1 mL of a 10 mM citric acid buffer solution (pH 6.0) with 8.77 mg of sodium chloride, 2.5 mg of phenol and 2.0 mg of Polysorbate 20 was antiseptically filled into a glass bottle. Each rubber stopper (rubber stopper B1 of Company B and rubber stoppers A1, A2, A3 and A4 of Company A) was immersed in the above-described sample, which was then stored at room temperature while shaking for a week. Thereafter, the metal ion concentration in the solution was measured.

### (iii) Experiment 3 Effects of Rubber Stopper on hGH

1 mL of a solution formed of 1 mL of a 10 mM citric acid buffer solution (pH 6.0) with 5.0 mg of hGH, 8.77 mg of sodium chloride, 2.5 mg of phenol and 2.0 mg of Polysorbate 20 was antiseptically filled into a glass bottle. A rubber stopper (rubber stopper B1 of Company B) with a high metal ion elution rate was immersed in the above-described sample, and 200 ppm of 2-sodium ethylene diamine 4-acetate was added. The change in the solubilization state was observed after letting stand at room temperature for 1 week.

### (iv) Experiment 4 Effects of Various Rubber Stoppers on hGH

1 mL of a solution formed of 1 mL of a 10 mM citric acid buffer solution (pH 6.0) with 5.0 mg of hGH, 8.77 mg of sodium chloride, 2.5 mg of phenol and 2.0 mg of Polysorbate 20 was antiseptically filled into a glass bottle. Each type of rubber stopper was immersed in the hGH solution. The pH change, content change, deamidate content and polymer content were measured after storing the prepared samples for one month under 5 °C and 25 °C conditions.

### 3) Experiment Results

### (i) Experiment 1 Effects of Metal Ions on hGH

With regard to zinc ions and aluminum ions, nebulation was observed in samples wherein 100 ppm and 50 ppm were respectively added. On the other hand, there was no nebulation in the samples into which magnesium ions and calcium ions were added (Table 1).

**Table 1**

| Metal Ion | Conc. Added | 0 ppm | 20 ppm | 50 ppm | 100 ppm |
|---|---|---|---|---|---|
| Zn²⁺ | Property pH | clear 6.06 | clear | clear 6.00 | nebulation 5.97 |
| Al³⁺ | Property pH | clear 6.03 | clear | nebulation 5.82 | nebulation 5.58 |
| Mg²⁺ | Property pH | clear 6.08 | clear | clear | clear |
| Ca²⁺ | Property pH | clear 6.05 | clear | clear | clear |

### (ii) Experiment 2 Elution of Metal lons from Rubber Stopper

With the rubber stopper B1 of Company B, the rate of elution of aluminum ions was considerably higher than in other rubber stoppers (Table 2).

**Table 2**

| Rubber Stopper | Zn²⁺ | Al³⁺ | Mg²⁺ | Ca²⁺ |
|---|---|---|---|---|
| B1 | 82.7 | 2.5 | 0.2 | 0.0 |
| A1 | 0.4 | 0.2 | 0.4 | 0.0 |
| A2* | 0.3 | 0.0 | 0.1 | 0.0 |
| A3* | 17.3 | 1.5 | 0.4 | 0.0 |
| A4* | 3.9 | 1.1 | 0.1 | 0.0 |

| | | | | |
|---|---|---|---|---|
| Note: elution units: ppm/unit | | | | |
| rubber stoppers weights approximately 850 mg/unit | | | | |
| *rubber stoppers weigh approximately 240 mg/unit | | | | |

### (iii) Experiment 3 Effects of Rubber Stoppers on hGH

The samples in which the rubber stopper B1 of company B were immersed were observed to have nebulation during storage. However, the sample in which a rubber stopper B1 of Company B was immersed after adding the chelating agent 2-sodium ethylene diamine 4-acetate was not observed to have nebulation (Table 3).

**Table 3**

| | No Rubber Stopper | Rubber Stopper Present* |
|---|---|---|
| EDTA 0 ppm | clear | nebulation |
| EDTA 200 ppm | clear | clear |

| | | |
|---|---|---|
| * rubber stopper B1 of Company B | | |

After one week of stationary storage at room temperature

### (iv) Experiment 4 Effects of Various Rubber Stoppers on hGH

Nebulation was observed during storage of a sample in which the rubber stopper B1 of Company B was immersed, and a drop in content was confirmed (25 °C for 1 month). Additionally, in the samples in which the rubber stopper B1 of Company B was immersed, the pH of the solution rose and there was considerable generation of deamidates and polymer content.

**Table 4**

| Rubber Stopper | Storage Conditions | pH | Solubility State | hGH Content¹⁾ | Deamidate Content²⁾ | Polymer Content |
|---|---|---|---|---|---|---|
| B1 | 5 °C for 1M | 6.72 | clear | 100% | 2.8% | 0.8% |
| | 25 °C for 1M | 7.75 | nebulation | 81% | 20.5% | 10.4% |
| A3 | 5 °C for 1M | 6.17 | clear | 99% | 2.8% | 0.3% |
| | 25 °C for 1M | 6.30 | clear | 102% | 10.7% | 1.0% |
| A4 | 5 °C for 1M | 6.20 | clear | 99% | 3.0% | 0.3% |
| | 25 °C for 1M | 6.41 | clear | 101% | 11.7% | 0.6% |
| A1 | 5 °C for 1M | 6.15 | clear | 100% | 3.0% | 0.2% |
| | 25 °C for 1M | 6.21 | clear | 100% | 10.7% | 0.5% |
| None | 5 °C for 1M | 6.08 | clear | 100% | 3.2% | 0.4% |

| | | | | | | |
|---|---|---|---|---|---|---|
| 1) The hGH content was calculated with the sample content after storage at 5 °C for 1 M without a stopper as 100%. | | | | | | |
| 2) The deamidate content includes cyclic imides. | | | | | | |

## Claims

1. A process for determining whether the material of a sealing member is such that the member is suitable for use in a storage container for a weakly acidic solution formulation containing human growth hormone, the process comprising the steps of:
(a) immersing the sealing member in 1 ml of a buffer solution containing a surfactant and having a pH of 5.5-6.5;
(b) storing the immersed sealing member at a temperature of 25°C for 1 week;
(c) simultaneously with step (b) shaking the immersed sealing member at a temperature of 25°C for 1 week; and
(d) measuring the elution rate of polyvalent metal ions in said buffer solution.

2. The process of claim 1 wherein step (d) is performed by atomic absorption spectrophotometry.

3. The process of claim 1 or 2 further comprising a step (e) of determining that the sealing member is suitable if the elution rate measured in step (d) is 50 ppm or less.

4. The process of claim 1 or 2 further comprising a step (e) of determining that the sealing member is suitable if the elution rate measured in step (d) is 20 ppm or less.

5. The process of any preceding claim including a step of using a sealing member of a material found to be suitable in a storage container for a weakly acidic solution formulation containing human growth hormone.

6. The process of claim 5 wherein said storage container comprises:
a cylindrical container having a first opening and a second opening, and an internal cavity connecting the first opening and second opening;
a first sealing member for sealing said first opening; and
a second sealing member provided in the internal cavity of said cylindrical container, such as to be capable of moving along said internal cavity while forming a continuous seal in a circumferential direction with an inner wall which forms this internal cavity, thereby forming an enclosed space with said first sealing member for containing the weakly acidic solution formulation containing human growth hormone; and wherein
said second sealing member is said sealing member of a material found to be suitable.

7. The process of claim 6 wherein said first sealing member is also composed of a said material.

8. The process of claim 6 or claim 7 wherein said storage container is an injection cartridge for a weakly acidic solution formulation containing human growth hormone, wherein said first sealing member for sealing said first opening has a thickness such as to be capable of being punctured by a syringe needle.

## Patentansprüche

1. Verfahren zur Bestimmung, ob das Material eines Dichtungselements solcherart ist, dass das Element zur Verwendung in einem Lagerungsbehälter für eine schwach saure Lösungsformulierung, die menschliches Wachstumshormon enthält, geeignet ist, wobei das Verfahren die folgenden Schritte umfasst:
(a) das Eintauchen des Dichtungselements in 1 ml einer Pufferlösung, die ein Tensid enthält und einen pH-Wert von 5,5 - 6,5 aufweist;
(b) das Lagern des eingetauchten Dichtungselements 1 Woche lang bei 25 °C;
(c) das gleichzeitig mit Schritt (b) durchgeführte Schütteln des eingetauchten Dichtungselements 1 Woche lang bei 25 °C; und
(d) das Messen der Elutionsrate mehrwertiger Metallionen in der Pufferlösung.

2. Verfahren nach Anspruch 1, worin Schritt (d) mithilfe der Atomabsorptions-Spektrophotometrie durchgeführt wird.

3. Verfahren nach Anspruch 1 oder 2, weiters umfassend einen Schritt (e) des Bestimmens, dass das Dichtungselement geeignet ist, wenn die in Schritt (d) gemessene Elutionsrate 50 ppm oder weniger beträgt.

4. Verfahren nach Anspruch 1 oder 2, weiters umfassend einen Schritt (e) des Bestimmens der Eignung des Dichtungselements, wenn die in Schritt (d) gemessene Elutionsrate 20 ppm oder weniger beträgt.

5. Verfahren nach einem der vorangegangenen Ansprüche, umfassend einen Schritt des Verwendens eines Dichtungselements aus einem Material, das als geeignet befunden wurde, in einem Lagerungsbehälter für eine schwach saure Lösungsformulierung, die menschliches Wachstumshormon enthält.

6. Verfahren nach Anspruch 5, worin der Lagerungsbehälter Folgendes umfasst:
einen zylindrischen Behälter mit einer ersten Öffnung und einer zweiten Öffnung sowie einem inneren Hohlraum, der die erste Öffnung und die zweite Öffnung verbindet;
ein erstes Dichtungselement zum Abdichten der ersten Öffnung; und
ein zweites Dichtungselement, das im inneren Hohlraum des zylindrischen Behälters so bereitgestellt ist, dass es zur Bewegung entlang dem inneren Hohlraum fähig ist, während es mit einer Innenwand, welche diesen inneren Hohlraum ausbildet, entlang der Umfangsrichtung eine durchgehende Abdichtung bildet, wodurch mit dem ersten Dichtungselement ein abgeschlossener Raum zur Aufnahme der schwach sauren Lösungsformulierung, die menschliches Wachstumshormon enthält, gebildet wird;
und worin es sich beim zweiten Dichtungselement um das Dichtungselement aus einem als geeignet befundenen Material handelt.

7. Verfahren nach Anspruch 6, worin das erste Dichtungselement ebenfalls aus besagtem Material besteht.

8. Verfahren nach Anspruch 6 oder 7, worin der Lagerungsbehälter eine Injektionskartusche für eine schwach saure Lösungsformulierung, die menschliches Wachstumshormon enthält, ist, worin das erste Dichtungselement zum Abdichten der ersten Öffnung eine Dicke aufweist, die das Durchstechen mit einer Spritzennadel zulässt.

## Revendications

1. Un procédé pour déterminer si la matière d'un élément d'étanchéité est telle que l'élément soit approprié pour être utilisé dans un récipient de stockage pour une préparation de solution faiblement acide contenant l'hormone de croissance humaine, le procédé comprenant les étapes consistant:
(a) immerger l'élément d'étanchéité dans 1 ml d'une solution tampon contenant un agent tensio-actif et présentant un pH de 5,5 à 6,5;
(b) stocker l'élément d'étanchéité immergé à une température de 25°C pendant 1 semaine;
(c) simultanément à l'étape (b) agiter l'élément d'étanchéité immergé à une température de 25°C pendant 1 semaine; et
(d) mesurer le taux d'élution des ions métalliques polyvalents dans ladite solution tampon.

2. Le procédé de la revendication 1 dans lequel l'étape (d) est effectuée par spectrophotométrie d'absorption atomique.

3. Le procédé de la revendication 1 ou 2 comprenant en outre une étape (e) consistant à déterminer que l'élément d'étanchéité est approprié si le taux d'élution mesuré dans l'étape (d) est de 50 ppm ou moins.

4. Le procédé de la revendication 1 ou 2 comprenant en outre une étape une étape (e) consistant à déterminer que l'élément d'étanchéité est approprié si le taux d'élution mesuré dans l'étape (d) est de 20 ppm ou moins.

5. Le procédé d'une quelconque revendication précédente comprenant une étape consistant à utiliser un élément d'étanchéité d'une matière trouvée pour être appropriée dans un récipient de stockage pour une préparation de solution faiblement acide contenant l'hormone de croissance humaine.

6. Le procédé de la revendication 5 dans lequel le récipient de stockage comprend:
un récipient cylindrique présentant une première ouverture et une seconde ouverture, ainsi qu'une cavité interne reliant la première ouverture et la seconde ouverture;
un premier élément d'étanchéité pour fermer de manière étanche ladite première ouverture; et
un second élément d'étanchéité prévu dans la cavité interne dudit récipient cylindrique, de telle manière à pouvoir se déplacer le long de ladite cavité interne tout en formant une étanchéité continue dans une direction circonférentielle avec une paroi interne qui délimite cette cavité interne, en délimitant ainsi un espace clos avec ledit premier élément d'étanchéité pour contenir la préparation de solution faiblement acide contenant l'hormone de croissance humaine; et où
ledit second élément d'étanchéité est ledit élément d'étanchéité d'une matière trouvée pour être appropriée.

7. Le procédé de la revendication 6 dans lequel ledit premier élément d'étanchéité est également composé d'une dite matière.

8. Le procédé de la revendication 6 ou de la revendication 7 dans lequel ledit récipient de stockage est une cartouche d'injection pour une préparation de solution faiblement acide contenant l'hormone de croissance humaine, où ledit premier élément d'étanchéité pour fermer de manière étanche la première ouverture présente une épaisseur telle qu'elle puisse être percée par l'aiguille d'une seringue.
